# EUROPEAN PATENT APPLICATION

(11) **EP 4 212 116 A1**
(43) Date of publication of application: **19.07.2023**
(21) Application number: 21875482.8
(22) Date of filing: 27.09.2021
(51) Int. Cl.: A61B 18/12

(54) **MEDICAL DEVICE AND METHOD OF CONTROLLING SAME**

(30) Priority: 29.09.2020 JP 2020163592
(71) Applicant: TERUMO Kabushiki Kaisha, Tokyo 151-0072 (JP)
(72) Inventor: FUKAMI, Kazunari, Ashigarakami-gun, Kanagawa 259-0151 (JP); TAKAHASHI, Yusuke, Ashigarakami-gun, Kanagawa 259-0151 (JP)
(74) Representative: Casalonga
(86) International application number: PCT/JP2021/035258
(87) International publication number: WO 2022/071180

(57) **Abstract**

A medical device configured to suppress a thrombus from being formed and a method for controlling the medical device are provided.

The medical device (10) according to the present embodiment includes: the expansion body (21) configured to expand and contract in a radial direction; the elongated shaft portion (20) including the distal portion (30) including the proximal end fixing portion (31) to which a proximal portion of the expansion body (21) is fixed; the plurality of electrodes (22) disposed along the expansion body (21); and the current supply unit (101) that supplies a current to the electrodes (22), in which the expansion body (21) includes the recess portion (51) recessed radially inward and defining the reception space (51b) configured to receive a biological tissue when the expansion body (21) expands, the plurality of electrodes (22) are arranged along the recess portion (51) to face the reception space (51b), and the current supply unit (101) is controlled to repeat supply and stop of a current to the electrodes (22) at least twice.

## Description

### Technical Field

The present invention relates to a medical device that applies energy to a biological tissue and a method for controlling the medical device.

### Background Art

Chronic heart failure is a known heart disease. Chronic heart failure is broadly classified into a systolic heart failure and a diastolic heart failure, based on a cardiac function index. In a patient suffering from the diastolic heart failure, myocardial hypertrophy appears, and stiffness (hardness) increases. Consequently, blood pressure increases in a left atrium, and a cardiac pumping function is degraded. In this manner, the patient may show heart failure symptoms such as a pulmonary edema. In addition, there is another heart disease of a patient who shows the following heart failure symptom. Due to pulmonary hypertension, blood pressure increases on a right atrium side, and the cardiac pumping function is degraded.

In recent years, shunt treatments have attracted attention. For the patients who suffer from heart failure, a shunt (puncture hole) serving as an escape route for increased atrial pressure is formed in an atrial septum, thereby enabling heart failure symptoms to be alleviated. In the shunt treatment, the atrial septum is accessed using an intravenous approaching method, and the puncture hole is formed to have a desired size.

In addition, Patent Literature 1 describes a device that cauterizes a biological tissue around an annulus part.

### Citation List

### Patent Literature

Patent Literature 1: JP 2010-68866 A

### Summary of Invention

### Technical Problem

When a high-frequency current is applied to an electrode, an atrial septum is cauterized, and the temperature of the electrode and the atrial septum increases. When the temperature reaches a predetermined temperature or greater, the blood near the electrode or the atrial septum starts to degenerate, and a thrombus is formed. When the thrombus is formed, the risk of occlusion of peripheral blood vessels arises as the thrombus is swept away by the blood.

The present invention has been made to solve the above-described problems, and an object is to provide a medical device configured to suppress a thrombus from being formed and a method for controlling the medical device.

### Solution to Problem

A medical device according to the present invention for achieving the above object includes: an expansion body configured to expand and contract in a radial direction; an elongated shaft portion including a distal portion including a proximal end fixing portion to which a proximal portion of the expansion body is fixed; a plurality of electrodes disposed along the expansion body; and a current supply unit that supplies a current to the electrodes, in which the expansion body includes a recess portion recessed radially inward and defining a reception space configured to receive a biological tissue when the expansion body expands, the plurality of electrodes are arranged along the recess portion to face the reception space, and the current supply unit is controlled to repeat supply and stop of a current to the electrodes at least twice.

A method for controlling a medical device according to the present invention for achieving the above object is a method for controlling a medical device including an expansion body configured to expand and contract in a radial direction including a recess portion recessed radially inward, an elongated shaft portion including a distal portion including a proximal end fixing portion to which a proximal portion of the expansion body is fixed, a plurality of electrodes disposed along a part of the recess portion of the expansion body, and a current supply unit that supplies a current to the electrodes, the method for controlling including: controlling the current supply unit to repeat, at least twice, supply and stop of a current supplied from the current supply unit to the electrodes.

### Advantageous Effects of Invention

Since the medical device configured as described above repeats the supply and stop of the current to the electrodes, it is possible to secure the cooling time of the electrodes and the biological tissue in contact with the electrodes to suppress a thrombus from being formed while maintaining the effect of cauterization by maintaining the total amount of energy output from the electrodes.

The current supply unit may be controlled such that the output of the electrodes decreases every time the current supply is repeated. This makes it possible to suppress the temperature of the electrode or the biological tissue from becoming equal to or greater than the blood degeneration temperature while considering accumulation of heat every time the repetition is performed.

The current supply unit may be controlled such that a cycle of starting supply of a current to the electrodes becomes shorter every time supply of a current is repeated. This allows the total amount of energy required for cauterization to be supplied in a short time, and the cauterization time to be shortened.

The current supply unit may be controlled such that the output of the electrodes becomes highest in the final supply of the supply of the current repeated a plurality of times. This allows the biological tissue to be strongly cauterized in the end, and therefore it becomes easy to hold, in a desired shape, the shape of the shunt to be formed.

The output time for continuing the supply of the current may be the shortest in the final supply. This makes it possible to suppress the temperature of the electrode or the biological tissue from becoming equal to or greater than the blood degeneration temperature by the final supply in which the output from the electrode is maximized.

At least one of the plurality of electrodes may be supplied with a current at a timing different from that of another electrode. This makes it possible to suppress the temperature of the electrode or the biological tissue from becoming equal to or greater than the blood degeneration temperature due to the difference in the heating timing by the electrode.

The expansion body may include a plurality of wire portions defining the recess portion to include a plurality of recess portions in which at least three of the recess portions are arranged at equal intervals in the circumferential direction of the expansion body, and the plurality of recess portions may each include the bottom portion, the proximal side upright portion, and the distal side upright portion. In this manner, since the recess portions are arranged at equal intervals in the circumferential direction of the expansion body, it is possible to form a shape close to a regular polygon when cauterizing the tissue around the puncture hole formed in the biological body, and it is possible to form a shunt having a size targeted by the operator.

The current supply unit may be controlled so that the current is supplied to the electrodes for 20 seconds or less per one time. This makes it possible to suppress the temperature of the electrode or the biological tissue from becoming equal to or greater than the blood degeneration temperature.

In the method for controlling the medical device configured as described above, since supply and stop of the current to the electrodes are repeated, it is possible to secure the cooling time of the electrodes and the biological tissue in contact with the electrodes to suppress a thrombus from being formed while maintaining the effect of cauterization by maintaining the total amount of energy output from the electrodes.

### Brief Description of Drawings

Fig. 1 is a front view illustrating an overall configuration of a medical device according to an embodiment.
Fig. 2 is an enlarged perspective view illustrating the vicinity of an expansion body.
Fig. 3 is an enlarged front view of the vicinity of the expansion body.
Fig. 4 is a view for schematically describing a state in which the expansion body is disposed in the atrial septum, in which the medical device is illustrated in a front view and the biological tissue is illustrated in a sectional view, respectively.
Fig. 5 is a view showing an expansion body stored in a storage sheath.
Fig. 6 is a view for schematically describing a state in which the expansion body is disposed in the right atrium, in which the medical device is illustrated in a front view and the biological tissue is illustrated in a sectional view, respectively.
Fig. 7 is a view for describing a state in which the diameter of the expansion body is increased in the atrial septum from the state of Fig. 6.
Fig. 8 is a view for schematically describing a state in which the expansion body is disposed in the atrial septum, in which the medical device is illustrated in a front view and the biological tissue is illustrated in a sectional view, respectively.
Fig. 9 is a graph showing a pattern of temporal changes in output from an electrode and electrode temperature.
Fig. 10 is a graph showing a modification example of the pattern of the temporal change in the output from the electrode and the electrode temperature.
Fig. 11 is a graph showing another modification example of the pattern of the temporal change in the output from the electrode and the electrode temperature.
Fig. 12 is a front view of the vicinity of an expansion body of a first modification example.
Fig. 13 is a front view of the vicinity of an expansion body of a second modification example.
Fig. 14 is a front view of the vicinity of an expansion body of a third modification example.
Fig. 15 is a front view of the vicinity of an expansion body of a fourth modification example.
Fig. 16 is a front view of the vicinity of an expansion body of a fifth modification example.
Fig. 17 is a front view of the vicinity of an expansion body of a sixth modification example.

### Description of Embodiment

Hereinafter, an embodiment of the present invention will be described with reference to the drawings. In some cases, dimensional ratios in the drawings may be exaggerated and different from actual ratios for convenience of description. In addition, in the present specification, a side on which a medical device 10 is inserted into a biological lumen will be referred to as a "distal end" or a "distal side", and an operating hand-side will be referred to as a "proximal end" or a "proximal side".

The medical device according to the embodiment described below is configured as follows. A puncture hole Hh formed in an atrial septum HA of a patient's heart H is enlarged, and further, a maintenance treatment is performed so that the puncture hole Hh having an increased diameter is maintained to have an increased size.

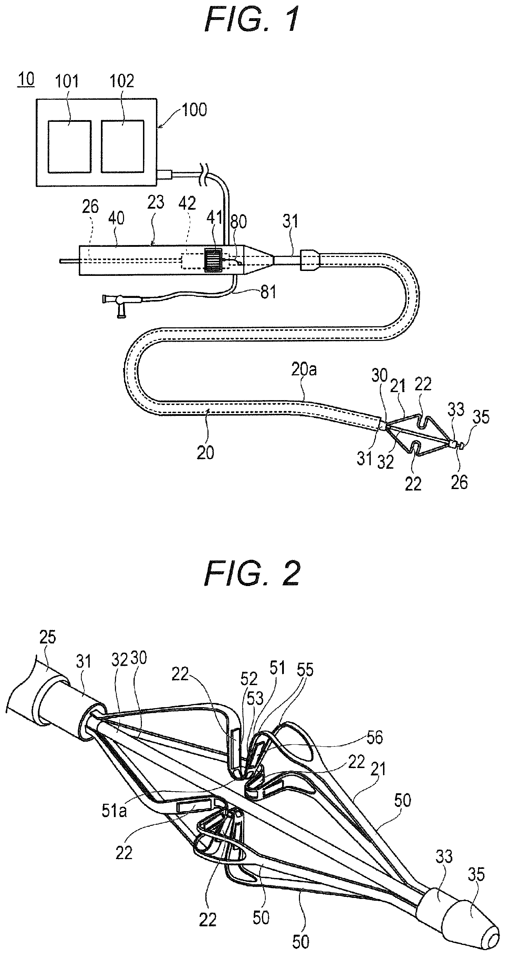

As illustrated in Figs. 1 to 3, the medical device 10 of the present embodiment includes an elongated shaft portion 20, an expansion body 21 disposed at a distal portion of the shaft portion 20, a plurality of electrodes 22, which are energy transfer elements for performing maintenance treatment, a hand operation unit 23 disposed at a proximal portion of the shaft portion 20, and an energy supply device 100.

The shaft portion 20 has a distal portion 30 including a proximal end fixing portion 31 to which a proximal end of the expansion body 21 is fixed and a distal end fixing portion 33 to which a distal end of the expansion body 21 is fixed. The distal portion 30 of the shaft portion 20 has a shaft extension portion 32 extending in the expansion body 21 from the proximal end fixing portion 31. The shaft portion 20 has a storage sheath 25 disposed at an outermost peripheral portion. The expansion body 21 is movable forward and rearward from the storage sheath 25 in an axial direction. In a state where the storage sheath 25 is moved to the distal side of the shaft portion 20, the storage sheath 25 can internally store the expansion body 21. In a state where the expansion body 21 is stored, the storage sheath 25 is moved to the proximal side. In this manner, the expansion body 21 can be exposed.

The shaft portion 20 includes a pulling shaft 26. The pulling shaft 26 is disposed from the proximal end of the shaft portion 20 to the shaft extension portion 32, and the distal portion is fixed to a distal member 35.

The distal member 35 to which the distal portion of the pulling shaft 26 is fixed needs not be fixed to the expansion body 21. In this manner, the distal member 35 can pull the expansion body 21 in a contracting direction. In addition, when the expansion body 21 is stored in the storage sheath 25, the distal member 35 is separated to the distal side from the expansion body 21. Accordingly, the expansion body 21 can be rather easily moved in an axial direction, and storage capability can be improved.

The hand operation unit 23 has a housing 40 configured to be held by an operator, an operation dial 41 that can be rotationally operated by the operator, and a conversion mechanism 42 operated in conjunction with the rotation of the operation dial 41. The pulling shaft 26 is held inside the hand operation unit 23 by the conversion mechanism 42. In conjunction with the rotation of the operation dial 41, the conversion mechanism 42 can move the held pulling shaft 26 forward and backward along the axial direction. For example, a rack and pinion mechanism can be used as the conversion mechanism 42.

The expansion body 21 has a plurality of wire portions 50 in a circumferential direction. In the present embodiment, for example, four of the wire portions 50 are disposed in the circumferential direction. The wire portions 50 are respectively configured to expand and contract in a radial direction. A proximal portion of the wire portion 50 extends to a distal side from the proximal end fixing portion 31. A distal portion of the wire portion 50 extends from a proximal portion to a proximal side of the distal end fixing portion 33. The wire portion 50 is inclined to increase in the radial direction from both end portions toward a central portion in an axial direction. In addition, in the wire portion 50, the central portion in the axial direction has a recess portion 51 recessed radially inward of the expansion body 21. A radially innermost portion of the recess portion 51 is a bottom portion 51a. The recess portion 51 defines a reception space 51b configured to receive a biological tissue when the expansion body 21 expands.

Each of the recess portions 51 includes a proximal side upright portion 52 extending radially outward from the proximal end of the bottom portion 51a and a distal side upright portion 53 extending radially outward from the distal end of the bottom portion 51a. The electrode 22 is disposed on the recess portion 51 so as to face the reception space 51b. In the distal side upright portion 53, a central portion in a width direction has a slit shape. The distal side upright portion 53 has an outer edge portion 55 on both sides and a backrest portion 56 of the central portion.

For example, the wire portion 50 forming the expansion body 21 has a flat plate shape cut out from a cylinder. The wire forming the expansion body 21 can have, for example, a thickness of a range of 50 µm to 500 µm and a width of a range of 0.3 mm to 2.0 mm. However, the wire may have a dimension outside this range. In addition, the wire portion 50 may have a circular shape in a cross section, or may have other shapes in a cross section.

Each of the electrodes 22 disposed in the wire portion 50 is disposed in the proximal side upright portion 52 so as to face the reception space 51b. The electrodes 22 may be disposed not in the proximal side upright portion 52 but in the distal side upright portion 53 so as to face the reception space 51b, or may be disposed in the bottom portion 51a so as to face the reception space 51b. Furthermore, the plurality of electrodes 22 may be arranged in each wire portion 50.

For example, a proximal side electrode 61 and a distal side electrode 62 are configured to include a bipolar electrode that receives electric energy from the energy supply device 100. In this case, electricity is supplied to the electrode 22. The electrode 22 and the energy supply device 100 are connected to each other by a conducting wire (not illustrated) coated with an insulating coating material. The conducting wire is drawn outward (i.e., extends) via the shaft portion 20 and the hand operation unit 23, and is connected to the energy supply device 100. The energy supply device 100 may be disposed in the hand operation unit 23.

Alternatively, the electrode 22 may be configured to serve as a monopolar electrode. In this case, the electricity is supplied from a counter electrode plate prepared outside a body. In addition, the electrode 22 may alternatively be a heating element (electrode chip) that generates heat by receiving high-frequency electric energy from the energy supply device 100. In this case, the electricity is supplied to the heating element. Furthermore, the electrode 22 can be configured to include an energy transfer element that applies energy to the puncture hole Hh, such as a heater including an electric wire which provides heating and cooling operation or generating frictional heat by using microwave energy, ultrasound energy, coherent light such as laser, a heated fluid, a cooled fluid, or a chemical medium. A specific form of the energy transfer element is not particularly limited.

The wire portion 50 is configured to be formed of a metal material. For example, the metal material can be a titanium-based (Ti-Ni, Ti-Pd, or Ti-Nb-Sn) alloy, a copperbased alloy, stainless steel, β-titanium steel, or a Co-Cr alloy. An alloy having a spring property such as a nickel titanium alloy may also be used as the material of the wire portion. However, a material of the wire portion 50 is not limited, and the wire portion 50 may be formed of other materials.

It is preferable that the shaft portion 20 is formed of a material having a certain degree of flexibility. For example, the materials of the shaft portion 20 may include polyolefin such as polyethylene, polypropylene, polybutene, ethylene-propylene copolymer, ethylene-vinyl acetate copolymer, ionomer, and a mixture of the above-described two or more materials, fluororesin such as soft polyvinyl chloride resin, polyamide, polyamide elastomer, polyester, polyester elastomer, polyurethane, and polytetrafluoroethylene, polyimide, PEEK, silicone rubber, or latex rubber.

For example, the pulling shaft 26 can be formed of the materials in which an elongated wire formed of a super elastic alloy such as a nickel-titanium alloy and a copperzinc alloy, a metal material such as stainless steel, or a resin material having relatively high rigidity is coated with a resin material such as polyvinyl chloride, polyethylene, polypropylene, and ethylene-propylene copolymer.

For example, the distal member 35 can be formed of a polymer material such as polyolefin, polyvinyl chloride, polyamide, polyamide elastomer, polyurethane, polyurethane elastomer, polyimide, and fluororesin or a mixture of polymer materials. Alternatively, the distal member 35 can be formed of a multilayer tube containing two or more polymer materials.

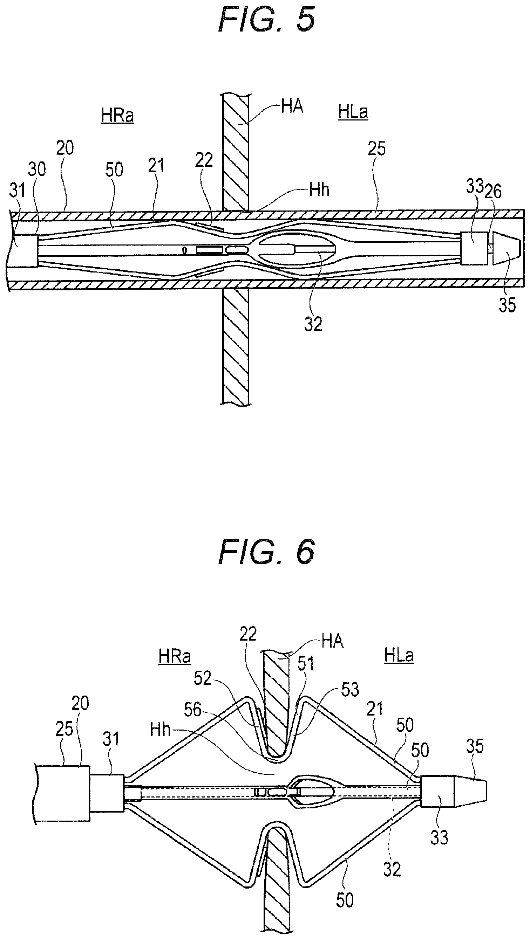

As shown in Fig. 5, the expansion body 21 housed in the storage sheath 25 is in a state of contracting in the radial direction. When the expansion body 21 and the storage sheath 25 move in the axial direction with respect to each other, the expansion body 21 is exposed outward of the storage sheath 25 and expands in the radial direction.

As illustrated in Fig. 1, the shaft portion 20 has a bent portion 20a bent in one direction in advance at a part proximal of the expansion body 21. This allows the operator to easily direct the distal end of the shaft 20 to the site of puncture of the atrial septum HA.

The hand operation unit 23 is provided with a display means for the operator to be able of grasp the orientation of the bent portion 20a inserted into the biological body. The hand operation unit 23 is provided with an orientation display unit 80 as the display means. The orientation display unit 80 displays a mark indicating the bending direction of the bent portion 20a, allowing the orientation of the shaft portion 20 inserted into the biological body to be recognized.

The hand operation unit 23 has a port 81 for priming the medical device 10. The direction in which the port 81 extends from the hand operation unit 23 is the same as the direction in which the bent portion 20a is bent. Since this also allows the operator to recognize the direction of the bent portion 20a, the port 81 may be used as the display means.

The energy supply device 100 has a current supply unit 101 that supplies a current to the plurality of electrodes 22 and a control unit 102 that controls the current supply unit 101. The control unit 102 includes, for example, a central processing unit (CPU), a storage circuit, and an operation program.

By controlling the current supply unit 101, the control unit 102 is configured to discretionarily adjust supply and stop of the high-frequency current from the current supply unit 101. The control unit 102 is configured to discretionarily adjust the output (power) of the high-frequency current from the current supply unit 101.

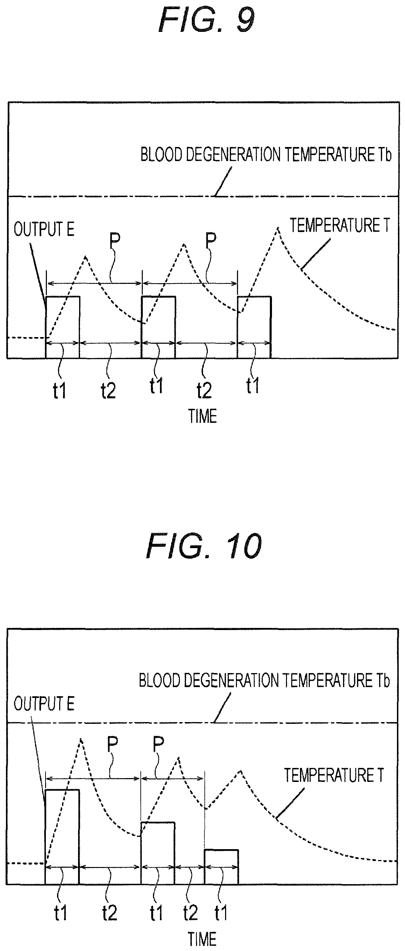

The current supply unit 101 is configured to supply a current to each of the electrodes 22. Under the control of the control unit 102, the current supply unit 101 is configured to start and stop the supply of the high-frequency current. The waveform of the high-frequency current supplied from the current supply unit 101 is a pulse shape (rectangular shape), but the waveform of the current is not limited to this. Although not particularly limited, the frequency of the high-frequency current is, for example, a range of 300 k to 115 MHz. As illustrated in Fig. 9, the current supply unit 101 is configured to repeat, twice or more, supply time t1 during which the high-frequency current is suppled. During the supply time t1 to be repeated, stop time t2 during which the high-frequency current is stopped is provided. The supply time t1 is preferably 20 seconds or less, more preferably a range of 0.5 seconds to 20 seconds, and still more preferably a range of 1 second to 15 seconds. This makes it possible to suppress temperature T of the electrodes 22 in contact with the blood and the biological tissue from becoming equal to or greater than blood degeneration temperature Tb at which degeneration of the blood appears. This makes it possible to suppress appearance of a thrombus caused by degeneration of blood in contact with the electrode 22 or the biological tissue. A cycle P in which the current supply unit 101 starts supplying the current to the electrodes 22 may be constant or may change. In addition, the stop time t2 may be constant or may change. The blood degeneration temperature Tb is about a range of 50 °C to 60 °C.

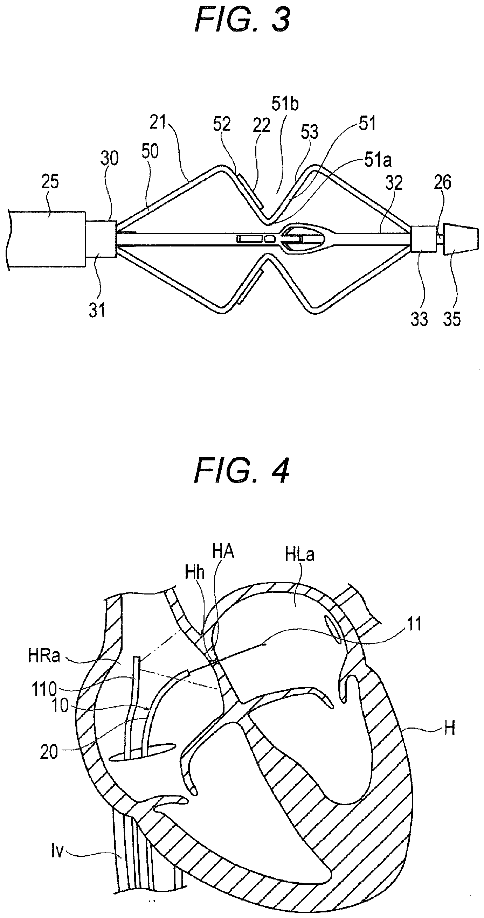

Next, a treatment method using the medical device 10 will be described. The treatment method according to the present embodiment is performed on a patient suffering from a heart failure (left heart failure). More specifically, as shown in Fig. 4, the treatment method is performed on the patient suffering from a chronic heart failure, who has high blood pressure in a left atrium HLa due to myocardial hypertrophy appearing in a left ventricle of the heart H and increased stiffness (hardness).

The treatment method according to the present embodiment includes forming the puncture hole Hh in the atrial septum HA (S1), disposing the expansion body 21 in the puncture hole Hh (S2), enlarging the diameter of the puncture hole Hh by using the expansion body 21 (S3), confirming hemodynamics in the vicinity of the puncture hole Hh (S4), performing the maintenance treatment for maintaining the size of the puncture hole Hh (S5), and confirming the hemodynamics in the vicinity of the puncture hole Hh after the maintenance treatment is performed (S6).

When the puncture hole Hh is formed, an operator delivers an introducer in which a guiding sheath and a dilator are combined with each other, to the vicinity of the atrial septum HA. For example, the introducer can be delivered to a right atrium HRa via an inferior vena cava Iv. In addition, the introducer can be delivered using the guide wire 11. The operator can insert the guide wire 11 into the dilator, and can deliver the introducer along the guide wire 11. The introducer and the guide wire 11 can be inserted into a living body by using a known method such as using a blood vessel introducer.

In the forming of the puncture hole Hh in the atrial septum HA (S1), the operator causes a puncture device (not illustrated) to penetrate from the right atrium HRa side toward the left atrium HLa side, thereby forming the puncture hole Hh. For example, a device such as a wire having a sharp distal end can be used as the puncture device. The puncture device is inserted into the dilator, and is delivered to the atrial septum HA. The puncture device can be delivered to the atrial septum HA instead of the guide wire 11 after the guide wire 11 is removed from the dilator.

In the enlarging of the diameter of the puncture hole Hh by using the expansion body 21 (S2), as illustrated in Fig. 4, the medical device 10 is first delivered to the vicinity of the atrial septum HA along the guide wire 11 inserted in advance. At this time, as illustrated in Fig. 5, the distal portion of the medical device 10 penetrates the atrial septum HA, and reaches the left atrium HLa. In addition, when the medical device 10 is inserted, the expansion body 21 is in a state of being stored in the storage sheath 25.

Next, the storage sheath 25 is moved to the proximal side so that the expansion body 21 is exposed. In this manner, as illustrated in Fig. 6, the diameter of the expansion body 21 increases, and the recess portion 51 is arranged in the puncture hole Hh of the atrial septum HA and receives the biological tissue surrounding the puncture hole Hh in the reception space 51b. The puncture hole Hh is maintained in a state of being expanded by the expansion body 21.

The shaft portion 20 of the medical device 10 is arranged such that the distal side is directed to the atrial septum HA in the right atrium HRa by the operator appropriately operating the shaft portion 20 while confirming the orientation of the bent portion 20a by the display means of the hand operation unit 23.

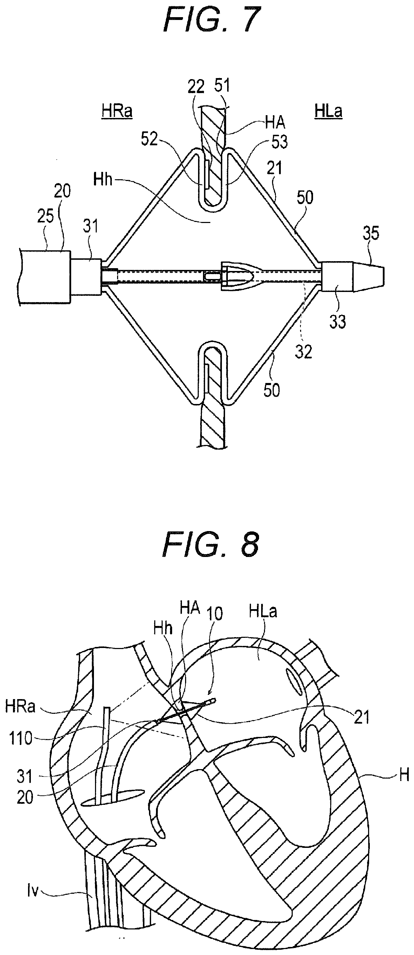

In the enlarging of the diameter of the puncture hole Hh by using the expansion body 21 (S3), the operator operates the hand operation unit 23 in a state where the recess portion 51 grips the atrial septum HA, and the pulling shaft 26 is moved to the proximal side, and sandwiches the biological tissue with the recess portion 51 of the expansion body 21 as illustrated in Fig. 7.

After the expansion body 21 is disposed in the puncture hole Hh, the hemodynamics is confirmed in the vicinity of the puncture hole Hh (S4). As shown in Fig. 8, the operator delivers a hemodynamics confirming device 110 to the right atrium HRa by way of the inferior vena cava Iv. For example, a known echo catheter can be used as the hemodynamics confirming device 110. The operator can display an echo image acquired by the hemodynamics confirming device 110 on a display apparatus such as a display, and can confirm a blood volume passing through the puncture hole Hh, based on a result of the echo image.

Next, the operator performs the maintenance treatment for maintaining the size of the puncture hole Hh (S5). In the maintenance treatment, high-frequency energy is applied to an edge portion of the puncture hole Hh through the electrode portion 22, thereby cauterizing (heating and cauterizing) the edge portion of the puncture hole Hh by using the high-frequency energy.

The operator operates the energy supply device 100 to start cauterization with each of the electrodes 22. The current supply unit 101 is controlled by the control unit 102 to repeat, at least twice, supply and stop of the high-frequency current to the electrodes 22. During the supply time t1 to be repeated, stop time t2 during which the high-frequency current is stopped is provided. For example, as in the example illustrated in Fig. 9, the current supply unit 101 repeats the constant supply time t1 twice or more. The supply time t1 is 20 seconds or less, and the cycle P is constant. Output E (power) from the electrodes 22 at each supply time t1 is substantially constant. The biological tissue supplied with the high-frequency current from the electrode 22 has an increased temperature by the internal resistance, and the electrodes 22 have an increased temperature by the heat transferred from the biological tissue in contact with the electrodes. When the supply time t1 is 20 seconds or less, it is possible to suppress the temperature T of the electrodes 22 in contact with blood and the biological tissue from becoming equal to or greater than the blood degeneration temperature Tb. At the stop time t2, the temperature T of the electrodes 22 in contact with blood and the biological tissue is cooled by the blood that is circulating. Since the temperature inside the biological tissue is not immediately cooled, the effect of cauterization can be favorably maintained. Then, by repeating the supply of the current from the current supply unit 101 with the stop time t2, it is possible to continue the cauterization of the biological tissue while suppressing the temperature T of the electrodes 22 in contact with blood and the biological tissue from becoming equal to or greater than the blood degeneration temperature Tb.

As in another example illustrated in Fig. 10, the current supply unit 101 may decrease the output E from the electrode 22 every time the current supply is repeated. Since the heat of the electrode 22 or the biological tissue is accumulated every time the supply of the current is repeated, the temperature T of the electrode 22 or the biological tissue easily becomes equal to or greater than the blood degeneration temperature Tb as the number of repetitions increases. Therefore, by decreasing the output E every time the current supply is repeated, it is possible to suppress the temperature T of the electrodes 22 in contact with blood and the biological tissue from becoming equal to or greater than the blood degeneration temperature Tb. As the number of repetitions increases and the output E from the electrode 22 decreases, the stop time t2, which is the cooling time, can be shortened, and thus the cycle P also decreases and the total energy required for cauterization (value obtained by integrating the output E (power) with time) can be supplied in a short time. Therefore, the cauterization time can be shortened.

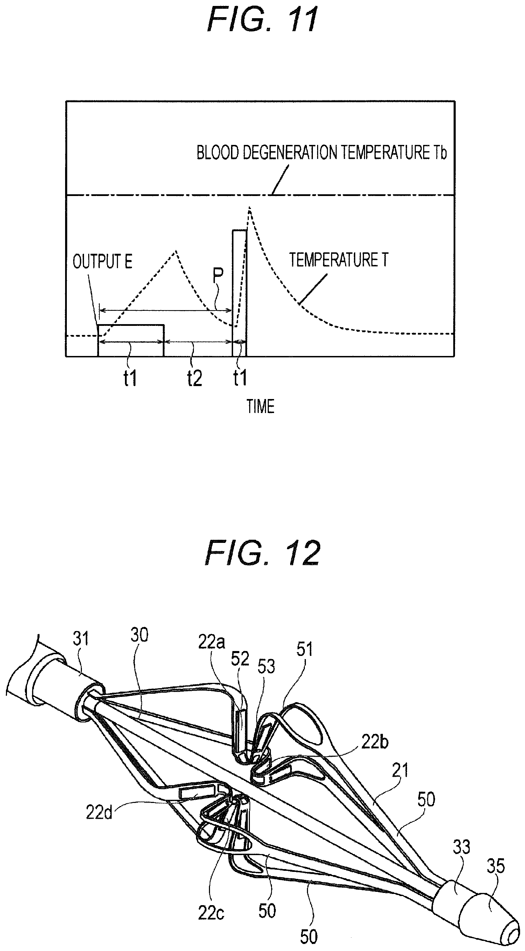

As in still another example illustrated in Fig. 11, the current supply unit 101 may decrease the output E of the first current supply and decrease the supply time t1 while increasing the output E in the final current supply. Increasing the output E allows the biological tissue to be strongly cauterized in the end, and therefore it becomes easy to hold, in a desired shape, the shape of the shunt to be formed. By decreasing the supply time t1, it is possible to suppress the temperature T of the electrodes 22 in contact with blood and the biological tissue from becoming equal to or greater than the blood degeneration temperature Tb by the final current supply in which the output E is maximized.

When the biological tissue in the vicinity of the edge portion of the puncture hole Hh is cauterized through the electrode portion 22, a degenerated portion having the degenerated biological tissue is formed in the vicinity of the edge portion. The biological tissue in the degenerated portion is in a state where elasticity is lost. Accordingly, the puncture hole Hh can maintain a shape widened by the expansion body 21.

After the maintenance treatment is performed, as illustrated in Fig. 8, the hemodynamics are confirmed again in the vicinity of the puncture hole Hh after the maintenance treatment (S6). In a case where the blood volume passing through the puncture hole Hh reaches a desired volume, the operator decreases the diameter of the expansion body 21. After the expansion body 21 is stored in the storage sheath 25, the expansion body 21 is removed from the puncture hole Hh. Furthermore, the whole medical device 10 is removed outward of the living body, and the treatment is completed.

In a case where the electrodes 22 are disposed not in the proximal side upright portion 52 but in the bottom portion 51a, the operator may perform cauterization in the state illustrated in Fig. 6 without sandwiching the biological tissue in the recess portion 51 of the expansion body 21 as illustrated in Fig. 7 in step S3.

As described above, the medical device 10 according to the present embodiment includes: the expansion body 21 configured to expand and contract in a radial direction; the elongated shaft portion 20 including the distal portion 30 including the proximal end fixing portion 31 to which a proximal portion of the expansion body 21 is fixed; the plurality of electrodes 22 disposed along the expansion body 21; and the current supply unit 101 that supplies a current to the electrodes 22, in which the expansion body 21 includes the recess portion 51 recessed radially inward and defining the reception space 51b configured to receive a biological tissue when the expansion body 21 expands, the plurality of electrodes 22 are arranged along the recess portion 51 to face the reception space 51b, and the current supply unit 101 is controlled to repeat supply and stop of a current to the electrodes 22 at least twice.

Since the medical device 10 configured as described above repeats the supply and stop of the current to the electrodes 22, it is possible to secure the cooling time of the electrodes 22 or the biological tissue in contact with the electrodes 22 to suppress a thrombus from being formed while maintaining the effect of cauterization by maintaining the total amount of energy output from the electrodes 22.

The current supply unit 101 may be controlled such that the output of the electrodes 22 decreases every time the current supply is repeated. This makes it possible to suppress the temperature of the electrode 22 or the biological tissue from becoming equal to or greater than the blood degeneration temperature Tb while considering accumulation of heat every time the repetition is performed. As the number of repetitions increases and the output from the electrode 22 decreases, the stop time t2 for cooling can also be shortened, and thus the cauterization time can be shortened.

The current supply unit 101 may be controlled such that a cycle P of starting supply of a current to the electrodes 22 becomes shorter every time supply of a current is repeated. This allows the total amount of energy required for cauterization to be supplied in a short time, and the cauterization time to be shortened.

The current supply unit 101 may be controlled such that the output of the electrodes 22 becomes highest in the final supply of the supply of the current repeated a plurality of times. This allows the biological tissue to be strongly cauterized in the end, and therefore it becomes easy to hold, in a desired shape, the shape of the shunt to be formed.

The output time t1 for continuing the supply of the current may be the shortest in the final supply. This makes it possible to suppress the temperature of the electrodes 22 or the biological tissue from becoming equal to or greater than the blood degeneration temperature Tb by the final supply in which the output E from the electrodes 22 is maximized.

The expansion body 21 may include a plurality of wire portions 50 defining the recess portion 51 to include a plurality of recess portions 51 in which at least three of the recess portions 51 are arranged at equal intervals in the circumferential direction of the expansion body 21, the plurality of recess portions 51 may each include the bottom portion 51a, the proximal side upright portion 52, and the distal side upright portion 53, and the plurality of electrodes 22 may be arranged in the recess portions 51 one by one. In this manner, since the recess portions 51 are arranged at equal intervals in the circumferential direction of the expansion body 21, it is possible to form a shape close to a regular polygon when cauterizing the tissue around the puncture hole Hh formed in the biological body, and it is possible to form a shunt having a size targeted by the operator.

The current supply unit 101 may be controlled so that the current is supplied to the electrodes 22 for 20 seconds or less per one time. This makes it possible to suppress the temperature of the electrodes 22 or the biological tissue from becoming equal to or greater than the blood degeneration temperature Tb.

The method for controlling the medical device 10 according to the present embodiment is a method for controlling the medical device 10 including the expansion body 21 configured to expand and contract in a radial direction including the recess portion 51 recessed radially inward, the elongated shaft portion 20 including the distal portion 30 including the proximal end fixing portion 31 to which a proximal portion of the expansion body 21 is fixed, the plurality of electrodes 22 disposed along a part of the recess portion 51 of the expansion body 21, and the current supply unit 101 that supplies a current to the electrodes 22, the method for controlling including: controlling the current supply unit 101 to repeat, at least twice, supply and stop of a current supplied from the current supply unit 101 to the electrodes 22. In the method for controlling configured as described above, since the current is not supplied to the electrodes 22 not in contact with the biological tissue of each of the electrodes 22 but only to the electrodes 22 in contact with the biological tissue, it is possible to suppress a thrombus from being formed and to effectively perform cauterization.

The present invention is not limited to the above-described embodiments, and various modifications can be made by those skilled in the art within the technical idea of the present invention. For example, it is not necessary for all the electrodes 22 to repeat supply and stop of the current at the same timing. As in the first modification example illustrated in Fig. 12, when four electrodes 22a, 22b, 22c, and 22d are disposed, the current supply for the electrode 22a and the electrode 22c is performed when the current supply for the electrode 22b and the electrode 22d is stopped, and the current supply for the electrode 22b and the electrode 22d is performed when the current supply for the electrode 22a and the electrode 22c is stopped. The number of electrodes 22 and the timing of repeating supply and stop of the high-frequency current are not limited. As described above, at least one of the plurality of electrodes 22a, 22b, 22c, and 22d may be supplied with the current at a timing different from that of the other electrodes. This makes it possible to suppress the temperature of the electrodes 22 or the biological tissue from becoming equal to or greater than the blood degeneration temperature Tb due to a difference in the heating timing by the electrodes 22.

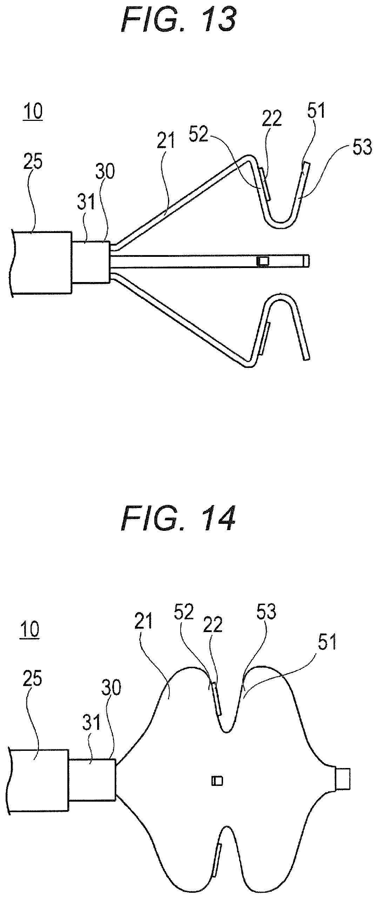

As in the second modification example illustrated in Fig. 13, the expansion body 21 may have a structure in which a site distal of the recess portion 51 is not disposed. The adjacent wire portions 50 are not coupled to each other in the example of Fig. 13, but may be coupled to each other.

As in the third modification example illustrated in Fig. 14, the expansion body 21 may be a balloon configured to expand when supplied with a fluid. The balloon is shaped to form the recess portion 51 when expanded.

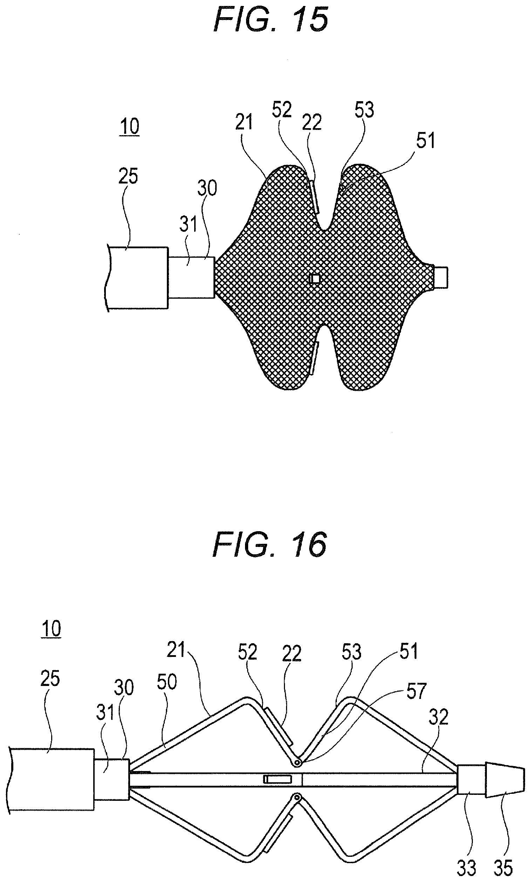

As in the fourth modification example illustrated in Fig. 15, the expansion body 21 may be formed of a mesh in which a large number of thin wires are knitted. The mesh is shaped to form the recess portion 51 when expanded.

As in the fifth modification example illustrated in Fig. 16, the expansion body 21 may be formed in a link structure coupled by a joint 57.

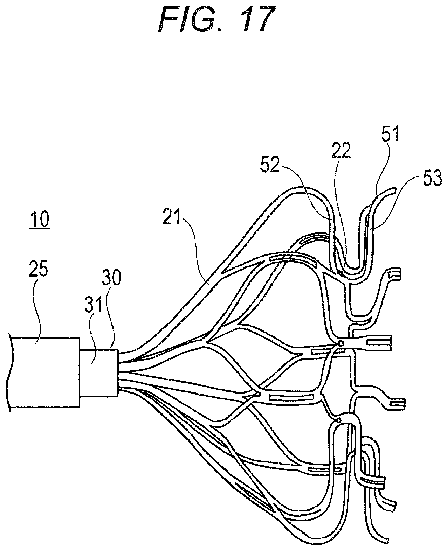

As in the sixth modification example illustrated in Fig. 17, the expansion body 21 may be formed in a mesh shape in which wire portions are branched and merged. The expansion body 21 has the plurality of recess portions 51, and the electrode 22 is disposed in the proximal side upright portion 52 of each of the recess portions 51. In the sixth modification example, the pulling shaft is not disposed. Therefore, the expansion body 21 released from the storage sheath 25 expands the puncture hole Hh only by its own expansion force.

The control unit 102 may receive temperature data from a temperature sensor (not illustrated) disposed in the vicinity of the electrodes 22, and perform feedback control of at least one of the supply time t1, the stop time t2, the output E, the cycle P, or the number of repetitions so that the temperature of the electrode 22 or the biological tissue does not become equal to or greater than the blood degeneration temperature Tb.

This application is based on Japanese Application No. 2020-163592 filed on September 29, 2020, the entire content of which is incorporated herein by reference.

### Reference Signs List

- 10: medical device
- 20: shaft portion
- 21: expansion body
- 22: electrode
- 30: distal portion
- 31: proximal end fixing portion
- 50: wire portion
- 100: energy supply device
- 101: current supply unit
- 102: control unit
- E: output (power)
- P: cycle
- T: temperature
- t1: supply time
- t2: stop time
- Tb: blood degeneration temperature

## Claims

1. A medical device comprising:
an expansion body configured to expand and contract in a radial direction;
an elongated shaft portion including a distal portion, the distal portion including a proximal end fixing portion to which a proximal portion of the expansion body is fixed;
a plurality of electrodes disposed along the expansion body; and
a current supply unit configured to supply a current to the electrodes, wherein
the expansion body includes a recess portion recessed radially inward and defining a reception space configured to receive a biological tissue when the expansion body expands,
the plurality of electrodes are arranged along the recess portion to face the reception space, and
the current supply unit is controlled to repeat supply and stop of a current to the electrodes at least twice.

2. The medical device according to claim 1, wherein the current supply unit is controlled in such a manner that output of the electrodes decreases every time the current supply is repeated.

3. The medical device according to claim 2, wherein the current supply unit is controlled in such a manner that a cycle of starting supply of a current to the electrodes becomes shorter every time the current supply is repeated.

4. The medical device according to claim 1, wherein the current supply unit is controlled in such a manner that output of the electrodes becomes highest in a final supply of the current supply repeated a plurality of times.

5. The medical device according to claim 4, wherein output time for continuing the current supply is shortest in the final supply.

6. The medical device according to any one of claims 1 to 5, wherein at least one of the plurality of electrodes is supplied with a current at a timing different from a timing of another electrode.

7. The medical device according to any one of claims 1 to 6, wherein
the expansion body includes a plurality of wire portions defining the recess portion to include a plurality of recess portions in which at least three of the recess portions are arranged at equal intervals in the circumferential direction of the expansion body, and
the plurality of recess portions each include the bottom portion, the proximal side upright portion, and the distal side upright portion.

8. The medical device according to any one of claims 1 to 7, wherein the current supply unit is controlled in such a manner that a current is supplied to the electrodes for 20 seconds or less per one time.

9. A method for controlling a medical device including an expansion body configured to expand and contract in a radial direction including a recess portion recessed radially inward, an elongated shaft portion including a distal portion, the distal portion including a proximal end fixing portion to which a proximal portion of the expansion body is fixed, a plurality of electrodes disposed along a part of the recess portion of the expansion body, and a current supply unit configured to supply a current to the electrodes, the method for controlling comprising:
controlling the current supply unit to repeat, at least twice, supplying and stopping of a current supplied from the current supply unit to the electrodes.

10. The method for controlling according to claim 9, wherein controlling the current supply unit in such a manner that current supply time per one time from the current supply unit to the electrode is 20 seconds or less.
